# EUROPEAN PATENT SPECIFICATION

(11) **EP 1 887 952 B1**
(45) Date of publication and mention of the grant of the patent: **11.02.2009**
(21) Application number: 06744401.8
(22) Date of filing: 24.05.2006
(51) Int. Cl.: A61B 17/66, A61B 17/64

(54) **Device for covered screwing of broken and shortened bones**
Vorrichtung zum verdeckten verschrauben von gebrochenen und verkürzten Knochen
Dispositif de vissage recouvert d'os fractures et raccourcis

(30) Priority: 24.05.2005 HU 0500111 U
(43) Date of publication of application: 20.02.2008
(73) Proprietor: Kadas, Istvan, 1037 Budapest (HU); Bagi, Istvan, 1116 Budapest (HU); Kadas, Daniel, 1037 Budapest (HU); Szita, Janos, 1118 Budapest (HU)
(72) Inventor: Kadas, Istvan, 1037 Budapest (HU); Bagi, Istvan, 1116 Budapest (HU); Kadas, Daniel, 1037 Budapest (HU); Szita, Janos, 1118 Budapest (HU)
(74) Representative: Erdély, Péter
(86) International application number: PCT/HU2006/000047
(87) International publication number: WO 2006/126033

(56) References cited:
- DE-A1- 4 300 369
- US-A- 4 308 863
- US-A1- 2003 212 400

## Description

Subject of the present invention is a device for covered screwing of broken and shortened bones - predominantly for heel bone fractures -, consisting of a distracting unit, targeting unit and bone screws.

Accurate alignment and compression of the fracture surface are basic premises of the healing of a broken bone. Osteosyntheses usually comply with these requirements. After injury of the internal frame, the limb shortens due to the traction effect of the muscles as bony fragments collapse. In the course of treatment, shortening effect of the muscles must be overpowered by contratraction, and broken surfaces must be aligned to each other in a closed or open manner. This is followed by the stabilization of the bony frame via plating, intramedullary nailing, external fixator or other known implants or devices, respectively.

The so called "ligamento-taxis" is frequently used in case of fractures involving the joints or located adjacent to them. The basic principle of this method is to achieve accurate reduction and fixation by the means of traction forces, exerted by ligaments and joint capsules attaching to the fragments, and transferring the force to the fragmented joint surface.

The heel bone is a sophisticated geometrical configuration, which is difficult to describe, as it has different processes, joint surfaces aligned in more planes, and is not similar to any ordinary geometrical profile. Thus, after injuries, resulting in multiple fractures, restoration and fixation is extremely difficult, when the joint surface is collapsed and the processes are broken. For that matter, predominantly open operative technique and plating used to be the preferable way of treatment in case of heel bone fractures with collapsed joint surface.

Nowadays, the so called "Zadravecz-method" became more widespread, performing the reduction in a closed manner. In the first step, distraction of the fragments is performed by the use of a distraction device, which is followed by the fixation of the fracture with three lag screws, applying minimally invasive operative technique. Today, this is the most popular and most frequently used way of fixation for fractures of the heel bone body, as this operative method requires an external fixation only during the intervention.

There is a kind of contradiction in this method, inasmuch as in the course of the same operation intra-operative restoration is achieved by traction, which is followed by application of compression in order to maintain the position. Further disadvantage of the closed method is that accurate targeting may only be aided by externally palpable processes.

Prior art document US-A-4 308 863 discloses an external fixation device composed of a pair of arcuate frame segments and multiple adjustment rods and pin holders, which cooperate with one another as well as plural pins extending through the soft tissue and bone of a patient to rigidly immobilize a bone fracture and permit compression, neutralization or distraction of the fracture from a location external to the body.

The object of the present invention is a device, which is suitable for distraction and fixation of the broken fragments in a desirably distracted position, achieved by external traction and application of special screws, and accurate screw positioning can be maintained during the intervention.

The device according to the invention consists of a distracting unit, targeting unit and special screws, where - according to our application for a patent - at least one of the screws is threaded at both ends. The thread close to the head of the screw has larger core diameter, thread diameter and thread increment, than those of the thread at the other end of the screw. The distraction device comprises three adjustment screws attached into an arched frame, where the terminal screws are coaxial, and enclose an angle of 90 degrees with the central screw. There are connecting elements at the internal ends of the screws, capable to accommodate K-wires. The targeting unit consists of an arched frame with one or more guiding sleeves at one end and a targeting spike on the other.

The screws may have bit heads and self-tapping threads. The outer diameter of the threads on the tip is preferably identical with the core diameter of the posterior threads.

The connecting elements of the adjustment screws on the distraction device are rotating eyes and at least one guiding sleeve and the targeting spike are arranged coaxially.

The most important intended use of the device constructed in this manner is the reduction and fixation of heel bone fractures.

Its main advantages are the simplification of the abolition of the shortening on one hand, and accurate and safe screw orientation during the closed intervention on the other. Further advantage is that closed technique is applicable, during which the stereo-tactic (spatial) targeting unit guides the screws in different planes. The direction of the traction may be altered by the use of the screws of the reduction device; desirable distraction can be achieved along the tips of a symmetrical triangle. Distractive effect of the screws provides with more accurate reduction. These objects are achieved by a device according to claim 1. Preferred embodiments are set forth in the dependent claims.

Further details of the invention will be explained by way of examples, with reference to the drawing, wherein
Fig. 1. shows a screw applied in the device according to the invention,
Fig. 2. is a plan view of the distraction unit according to the invention,
Fig. 3. is a lateral view of an advantageous embodiment of the targeting unit,
Fig. 4. is a plan view of another advantageous embodiment of the targeting unit and
Fig. 5. shows the reduction of a heel bone from two perpendicular views.

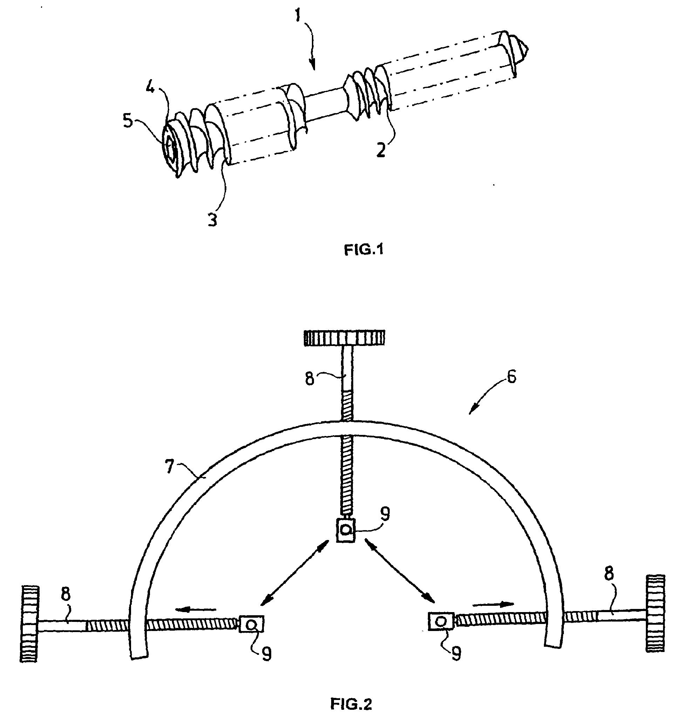

The distraction screw (1) shown in Figure 1 is provided with threads (2 and 3) on both ends. Both threads are self-tapping in the direction of insertion. The diameter of the thread (2) next to the tip of the screw (1) is 4.5 mm, the pitch is 1 mm, while the thread (3), adjacent to the screw head (4), is 6.5 mm in diameter and the pitch is 1.5 mm. This means that the both the pitch and diameter of the threads next to the screw tip are smaller, than those of the posterior thread (3). In this embodiment, the outer diameter of the anterior thread (2) is identical with the core diameter of the posterior thread (3). Thus, insertion and rotation of the two threads (2 and 3) of the distraction screw (1) into two broken bone fragments results in distraction between the two segments. This effect is utilized in the course of closed screw-fixation of heel bone fractures. There is an inner wrench opening (5) on the head (4) of the screw.

According to the invention, the reduction of the broken heel bone begins with the use of a distracting unit (6), applied in triangular position, corresponding to the anatomy of the calcaneus. Figure 2 schematically demonstrates the distracting unit (6). There are threaded bores in a semicircular frame (7), in 0-90-180 degrees position, accommodating adjustment screws (8). There are connecting elements (9) at the ends of the adjustment screws (8) for fixation of K-wires drilled into the bone fragments, and for repositioning said bone fragments. The connecting elements (9) of the adjustment screws (8) are rotating eyes in this embodiment.

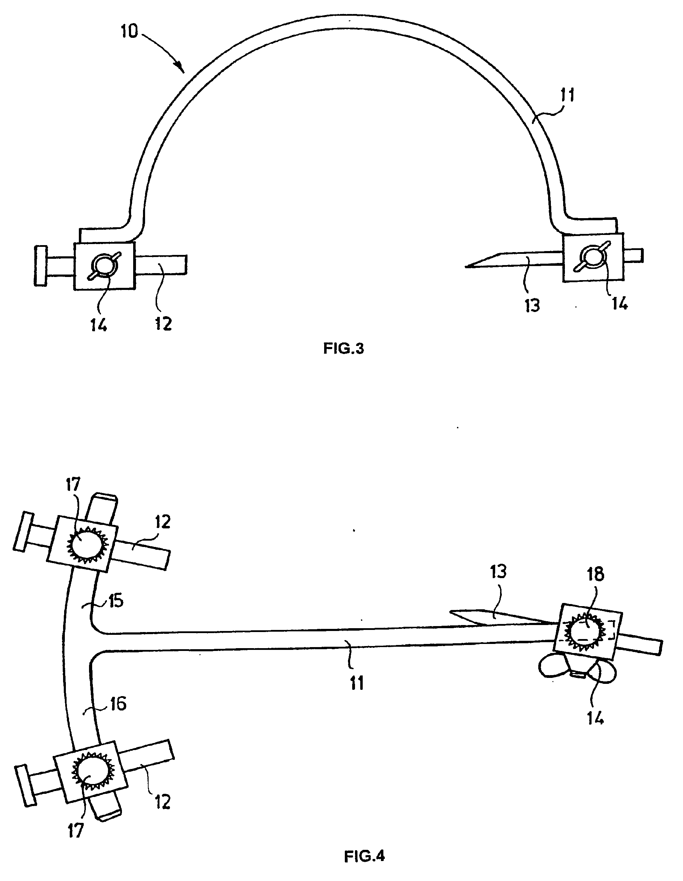

The targeting unit (10), as shown in Figure 3., is used for appropriate screw insertion and positioning. This device - similarly to the distracting unit (6) - contains a bended frame (11). There is a guiding sleeve (12) attached to the one end of the frame, and a targeting spike (13) on the opposite end of the frame (11), in coaxial position. Both guiding sleeve (12) and targeting spike (13) are longitudinally adjustable and can be fixed with clamping screws (14).

The targeting unit (10) may optionally be equipped with more guiding sleeves (12) (and with more targeting spikes too, if desired). Figure 4. demonstrates an embodiment with more guiding sleeves (12). Guiding sleeves (12) are arranged on branches (15 and 16) of the frame (11), and may be fixed with clamping screws (17). The targeting spike (13) is preferably rotatable around its longitudinal axis and may be fixed with a clamping screw (18), keeping it always coaxially with the corresponding guiding sleeve (12).

The device according to the invention may be used in the restoration of all fractures, where broken bone fragments are contracted by the muscles and shortening occurs. Such fracture patterns are typically observed in case of heel bone, ankle or wrist fractures. Figure 4. demonstrates the use of the device in case of the reduction of a heel bone fracture.

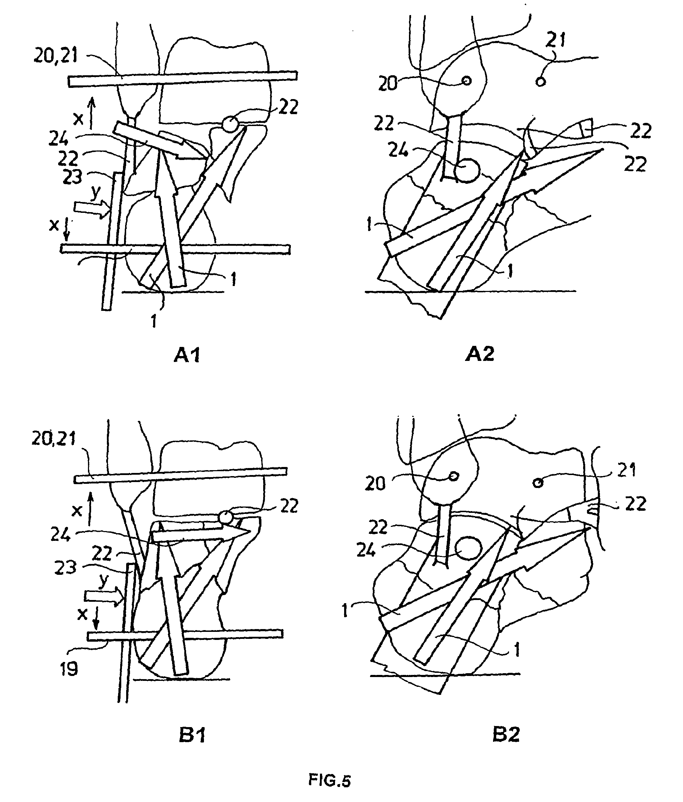

Segment A1 of Figure 5. shows a broken heel bone in rear view, meanwhile segment A2 shows it in side view. The restored bones are shown likewise in segments B1 and B2 in rear view and side view, respectively..

According to the anatomical situation, restoration of the heel bone is achieved by a distracting unit (6), positioned in a triangular arrangement. The wire (19), positioned transversally into the heel bone, adjacent to the Achilles tendon, makes up one tip of the triangle (the sharpened tip wire is a rigid, straight K-wire, a so called Kirschner-wire) and wires (20 and 21), also drilled transversally into the neighboring bones, make up the other two tips of the triangle. Desirable distraction and reduction of the bone fragments is achieved by the distraction of the wires (19, 20 and 21) along axis "X", and against the force arising from the muscles, while lateral pressure is exerted to the displaced bone fragments, along axis Y (this is achieved by hammer strikes, exerted on an intermediate plate (23)).

Having achieved the desired position of the bone fragments by the use of the distracting unit (6), bores are drilled into the irregular shaped heel bone for the screws, also making up a triangle. For this purpose, the embodiment or the targeting unit (10) demonstrated on Figure 4 is used, applying the easily palpable and verifiable projection of the heel bone (sustentaculum tali) under the internal ankle for positioning the targeting spike (13) of the targeting unit (10). In this manner, the guiding sleeve (12) aims the drill bit towards the targeting spike (13), 1.5 cm-s under and behind the external ankle.

The second bore is prepared obliquely towards the tip of the targeting unit (10). The third bore is positioned along the longitudinal axis of the heel bone, aiming internally and upwards from the second one.

Once the bores are prepared, a traditional double-threaded compression screw (24) is inserted transversally. This screw stabilizes the broken fragments.

The second screw distracts the fragments, in order to abolish shortening, arising from the fracture. For this purpose, the double threaded, distraction screw (1) of Fig. 1. is used. As shown on the figure, the third screw is positioned internally and upwards from the second one. This third screw is positioned in the longitudinal axis of the heel bone, supporting the collapsed and restored joint surface. This screw is responsible for maintaining the length, thus, as a consequence, a double-threaded, distracting screw (1) is needed for this purpose as well.

By the use of the targeting unit according to the invention, may be assure that the screws inserted in the course of the closed reduction do not cross each other, i.e. they are applied in different planes.

Furthermore, the distraction screws together with the distracting unit, not used so far, are capable to distract the reduced bone fragments, overpowering the traction force of the muscles.

In this way, the use of the device according to the invention makes closed reduction of shortened fractures considerably easier and safer and strongly reduces inconvenience to the patient arising from the operation and helps prevent complications.

Though the invention was described with reference to a heel bone fracture, the device may obviously be used in case of other fractures, too. It is also obvious that the design of the device as well as the screws and units applied therein may differ from those demonstrated in the example and many other constructions are also feasible within the scope claimed in the attached patent claims.

## Claims

1. A device for screw-stabilization of shortened fractures, mainly heel bone fractures, consisting of a distracting unit (6), a targeting unit (10) and screws,
**characterized in that**
• at least one screw is a distraction screw (1) with threads (2, 3) at both ends; wherein core diameter, thread diameter and pitch of the thread (3) next to the head (4) of the screw are greater than those parameters of the anterior thread (2);
• three adjustment screws (8) are attached to a bended frame (7) of the distracting unit (6), wherein the terminal adjustment screws are coaxial and enclose an angle of 90 degrees with the central screw, and there are connecting elements (9) at the internal ends of the screws, capable to accommodate K-wires (19, 20, 21);
• the targeting unit (10) consists of a bended frame (11) equipped with a targeting spike (13) on one end, and with one or more guiding sleeves (12) on the other end.

2. The device as claimed in claim 1, **characterized in that** the distraction screws (1) are provided with bit heads.

3. The device as claimed in claim 1 or 2, **characterized in that** the threads of the distraction screws (1) are self-tapping threads (2, 3).

4. The device as claimed in any of claims 1 to 3, **characterized in that** the outer diameter of the anterior thread (3) of the distraction screw (1) is identical with the core diameter of the posterior thread (2).

5. The device as claimed in any of claims 1 to 4, **characterized in that** the connecting elements (9) of the adjustment screws (8) on the distracting unit (6) are rotating eyes.

6. The device as claimed in any of claims 1 to 5, **characterized in that** there is a guiding sleeve (12) on the targeting unit (10), said sleeve being coaxial with the targeting spike (13).

7. The device as claimed in any of claims 1 to 5, **characterized in that** there are more guiding sleeves (12) on the targeting unit (10), and one of them is coaxial with the targeting spike (13).

8. The device as claimed in claim 7, **characterized in that** there are more guiding sleeves (12) arranged on the branches (15, 16) of the bended frame (11).

9. The device as claimed in claim 8, **characterized in that** it is rotatable and fixable in a plane perpendicular to the longitudinal axis of the targeting spike (13).

## Patentansprüche

1. Einrichtung zur mittels Verschraubung erfolgender Stabilisierung von mit Verkürzung einhergehenden Brüchen, insbesondere Fersenbeinbrüchen, welche eine Distraktionseinheit, eine Zieleinheit und Schrauben umfasst, **dadurch gekennzeichnet, dass**
• mindestens eine Schraube eine Distraktionsschraube (1) mit an beiden Enden vorgesehenen Gewinden (2 und 3) ist; wobei der Kerndurchmesser, der Gewindedurchmesser und die Gewindesteigung des zu dem Kopf (4) der Schraube näher befindlichen Gewindes (2) größer als diese Parameter des vorderen Gewindes (3) sind;
• drei Stellschrauben (8) an einem gebogenen Rahmen (7) der Distraktionseinheit (6) angebracht sind, wobei die äußeren Stellschrauben gleichachsig angeordnet sind und mit der zentralen Schraube einen Winkel von 90° einschließen, und an den inneren Enden der Schrauben Verbindungselemente (9) zur Aufnahme von K-Drähten (19, 20, 21) vorgesehen sind;
• die Zieleinheit (10) einen gebogenen Rahmen (11) aufweist, welcher an einem Ende mit einem Zieldorn (13) versehen ist, und an dem anderen Ende eine oder mehrere Führungshülsen (12) aufweist.

2. Einrichtung nach Anspruch 1, **dadurch gekennzeichnet, dass** die Distraktionsschrauben (1) mit Bohrspitzen versehen sind.

3. Einrichtung nach Anspruch 1 oder 2, **dadurch gekennzeichnet, dass** die Gewinde der Distraktionsschrauben (1) selbstschneidende Gewinde (2, 3) sind.

4. Einrichtung nach einem der Ansprüche 1 bis 3, **dadurch gekennzeichnet, dass** der Aussendurchmesser des vorderen Gewindes (3) der Distraktionsschraube (1) mit dem Kerndurchmesser des hinteren Gewindes (2) identisch ist.

5. Einrichtung nach einem der Ansprüche 1 bis 4, **dadurch gekennzeichnet, dass** die Verbindungselemente (9) der Stellschrauben (8) auf der Distraktionseinheit (6) rotierende Ösen sind.

6. Einrichtung nach einem der Ansprüche 1 bis 5, **dadurch gekennzeichnet, dass** auf der Zieleinheit (10) eine Führungshülse (12) vorgesehen ist, wobei die Hülse zu dem Zieldorn (13) koaxial angeordnet ist.

7. Einrichtung nach einem der Ansprüche 1 bis 5, **dadurch gekennzeichnet, dass** mehrere Führungshülsen (12) auf der Zieleinheit (10) vorgesehen sind, wobei eine von diesen zu dem Zieldorn (13) koaxial angeordnet ist.

8. Einrichtung nach Anspruch 7, **dadurch gekennzeichnet, dass** mehrere Führungshülsen (12) auf den Schenkeln (15, 16) des gebogenen Rahmens (11) angeordnet sind.

9. Einrichtung nach Anspruch 8, **dadurch gekennzeichnet, dass** sie in einer zur Längsachse des Zieldorns (13) senkrechten Ebene drehbar und fixierbar angeordnet ist.

## Revendications

1. Dispositif pour une stabilisation par vis de fractures réduites, essentiellement des fractures des os du talon, consistant en une unité de distraction (6), une unité de ciblage (10) et des vis, **caractérisé en ce que**
• au moins une vis est une vis de distraction (1) avec des filets (2, 3) aux deux extrémités ; dans laquelle, diamètre d'âme, diamètre de filet et pas de filet (3) à proximité de la tête (4) de la vis sont plus grands que ces paramètres du filet antérieur (2) ;
• trois vis d'ajustement (8) sont fixées à un cadre arqué (7) de l'unité de distraction (6), dans lequel les vis d'ajustement terminales sont coaxiales et constituent un angle de 90° avec la vis centrale, et il y a des éléments de connexion (9) aux extrémités internes des vis, aptes à recevoir des fils de Kirschner (19, 20, 21) ;
• l'unité de ciblage (10) consiste en un cadre arqué (11) équipé avec une pointe de ciblage (13) sur une extrémité, et avec un ou plusieurs manchons de guidage (12) sur l'autre extrémité.

2. Dispositif selon la revendication 1, **caractérisé en ce que** les vis de distraction (1) sont prévues avec des têtes de foret.

3. Dispositif selon la revendication 1 ou 2, **caractérisé en ce que** les filets des vis de distraction (1) sont des filets autotaraudeurs (2, 3).

4. Dispositif selon l'une quelconque des revendications 1 à 3, **caractérisé en ce que** le diamètre extérieur du filet antérieur (3) de la vis de distraction (1) est identique au diamètre d'âme du filet postérieur (2).

5. Dispositif selon l'une quelconque des revendications 1 à 4, **caractérisé en ce que** les éléments de connexion (9) des vis d'ajustement (8) sur l'unité de distraction (6) sont des oeillets rotatifs.

6. Dispositif selon l'une quelconque des revendications 1 à 5, **caractérisé en ce qu'**il y a un manchon de guidage (12) sur l'unité de ciblage (10), ledit manchon étant coaxial avec la pointe de ciblage (13).

7. Dispositif selon l'une quelconque des revendications 1 à 5, **caractérisé en ce qu'**il y a plusieurs manchons de guidage (12) sur l'unité de ciblage (10), et l'un d'entre eux est coaxial avec la pointe de ciblage (13).

8. Dispositif selon la revendication 7, **caractérisé en ce qu'**il y a plusieurs manchons de guidage (12) agencés sur les branches (15, 16) du cadre arqué (11).

9. Dispositif selon la revendication 8, **caractérisé en ce qu'**il est rotatif et fixable dans un plan perpendiculaire à l'axe longitudinal de la pointe de ciblage (13).
